# EUROPEAN PATENT SPECIFICATION

(11) **EP 2 288 367 B1**
(45) Date of publication and mention of the grant of the patent: **07.10.2015**
(21) Application number: 09747742.6
(22) Date of filing: 15.05.2009
(51) Int. Cl.: A61K 38/22, A61P 15/06

(54) **H2 relaxin for use in the treatment of premature cervical dilation**
H2-Relaxin zur Verwendung bei der Behandlung von vorzeitiger Zervixdilatation
Relaxine H2 pour son utilisation dans le traitement de la dilatation prématurée du col utérin

(30) Priority: 16.05.2008 US 127888 P
(43) Date of publication of application: 02.03.2011
(73) Proprietor: Corthera, Inc., San Mateo, CA 94402 (US)
(72) Inventor: STEWART, Dennis, R., Los Gatos, CA 95033 (US)
(74) Representative: Westberg, Marie Suzanne
(86) International application number: PCT/US2009/044251
(87) International publication number: WO 2009/140661

(56) References cited:
- US-A- 4 758 516
- US-A- 5 545 616
- US-A- 5 707 642
- US-A- 5 707 642
- US-A- 6 075 005
- US-A1- 2002 031 513
- US-A1- 2002 031 513
- US-A1- 2006 247 172
- US-A1- 2006 247 172
- "Preterm labor" In: Anonymous: "The merck manual of diagnosis and therapy. 18th edition", 2006, Merck Research Laboratories, Whitehouse Station, NJ, XP002695988, ISBN: 0911910182 pages 2205-2205, * the whole document *
- G. D. BRYANT-GREENWOOD ET AL: "Human Decidual Relaxin and Preterm Birth", ANNALS OF THE NEW YORK ACADEMY OF SCIENCES, vol. 1041, no. 1, 1 May 2005 (2005-05-01), pages 338-344, XP055060923, ISSN: 0077-8923, DOI: 10.1196/annals.1282.054
- AKINBAMI M A ET AL: "Cervical dilation, conception rate, and concentrations of progesterone and estradiol-17B in postpartum ewes treated with porcine relaxin", THERIOGENOLOGY, LOS ALTOS, CA, US, vol. 34, no. 5, 1 November 1990 (1990-11-01), pages 927-940, XP023189924, ISSN: 0093-691X, DOI: 10.1016/0093-691X(90)90562-8 [retrieved on 1990-11-01]
- Anonymous: "Premature birth", Mayo clinic , Retrieved from the Internet: URL:http://www.mayoclinic.org/diseases-con ditions/premature-birth/basics/risk-factor s/con-20020050?p=1

### SEQUENCE LISTING

<110> STEWART, Dennis R.
<120> METHOD OF PREVENTING PREMATURE DELIVERY
<130> 64325-2001240
<140> PCT/US2009/044251
   <141> 2009-05-15
<150> US 61/127,888
   <151> 2008-05-16
<160> 8
<170> FastSEQ for Windows Version 4.0
<210> 1
   <211> 29
   <212> PRT
   <213> Homo sapiens
<400> 1
   Asp Ser Trp Met Glu Glu Val Ile Lys Leu Cys Gly Arg Glu Leu Val
    1               5                  10                  15
   Arg Ala Gln Ile Ala Ile Cys Gly Met Ser Thr Trp Ser
               20                  25
<210> 2
   <211> 24
   <212> PRT
   <213> Homo sapiens
<220>
   <221> VARIANT
   <222> (1)...(24)
   <223> Xaa = Glu or Gln
<400> 2
   Xaa Leu Tyr Ser Ala Leu Ala Asn Lys Cys Cys His Val Gly Cys Thr
    1               5                  10                  15
   Lys Arg Ser Leu Ala Arg Phe Cys
               20
<210> 3
   <211> 8
   <212> PRT
   <213> Artificial Sequence
<220>
   <223> Relaxin consensus sequence
<221> VARIANT
   <222> (1)...(8)
   <223> Xaa = Any Amino Acid
<400> 3
   Arg Glu Leu Val Arg Xaa Xaa Ile
    1               5
<210> 4
   <211> 33
   <212> PRT
   <213> Artificial Sequence
<220>
   <223> Synthesized Construct
<221> VARIANT
   <222> 1
   <223> Xaa = Absent or Gly or a small naturally or
   non-naturally occurring amino acid
<221> VARIANT
   <222> 2
   <223> Xaa = Absent or Gln or a polar naturally or
   non-naturally occurring amino acid
<221> VARIANT
   <222> 3
   <223> Xaa = Absent or Lys or a basic naturally or
   non-naturally occurring amino acid
<221> VARIANT
   <222> 4
   <223> Xaa = Absent or Gly or a small naturally or
   non-naturally occurring amino acid
<221> VARIANT
   <222> 5
   <223> Xaa = Absent or Gln or Ser or a polar naturally or
   non-naturally occurring amino acid
<221> VARIANT
   <222> 6
   <223> Xaa = Absent or Val or Ala or Pro or Met or
   a hydrophobic naturally or non-naturally occurring amino acid
<221> VARIANT
   <222> 7
   <223> Xaa = Absent or Gly or a small naturally or
   non-naturally occurring amino acid
<221> VARIANT
   <222> 8
   <223> Xaa = Absent or Pro or Leu or Ala or a naturally or
   non-naturally occurring amino acid
<221> VARIANT
   <222> 9
   <223> Xaa = Absent or Pro or Gln or a naturally or
   non-naturally occurring amino acid
<221> VARIANT
   <222> 10
   <223> Xaa = Absent or Gly or a small naturally or
   non-naturally occurring amino acid
<221> VARIANT
   <222> 11
   <223> Xaa = Absent or Ala or His or Glu or Asp or
   a hydrophobic or a small or an acidic naturally or
   non-naturally occurring amino acid
<221> VARIANT
   <222> 12
   <223> Xaa = Absent or Ala or Pro or Gln or Ser or Arg or
   His or a hydrophobic or a small naturally or non- naturally
   occurring amino acid
<221> VARIANT
   <222> 13
   <223> Xaa = Absent or Cys or Val or a hydrophobic naturally or
   non-naturally
   occurring amino acid
<221> VARIANT
   <222> 14
   <223> Xaa = Absent or Arg or Lys or Gln or Pro or a basic or
   a polar naturally or non-naturally occurring amino acid
<221> VARIANT
   <222> 15
   <223> Xaa = Absent or Arg or Gln or Ser or a basic or a polar
   naturally or non-naturally occurring amino acid
<221> VARIANT
   <222> 16
   <223> Xaa = Absent or Ala or Leu or His or Gln or a hydrophobic
   or a small naturally or non-naturally occurring amino acid
<221> VARIANT
   <222> 17
   <223> Xaa = Absent or Tyr or a hydrophobic or an aromatic
   naturally or non-naturally occurring amino acid
<221> VARIANT
   <222> 18
   <223> Xaa = Absent or Ala or a hydrophobic or small naturally
   or non-naturally occurring amino acid
<221> VARIANT
   <222> 19
   <223> Xaa = Absent or Ala or a hydrophobic small naturally or
   non-naturally occurring amino acid
<221> VARIANT
   <222> 20
   <223> Xaa = Absent or Phe or a hydrophobic or an aromatic
   naturally or non-naturally occurring amino acid
<221> VARIANT
   <222> 21
   <223> Xaa = Absent or Ser or Thr or a polar naturally or
   non-naturally occurring amino acid
<221> VARIANT
   <222> 22
   <223> Xaa = Absent or Val or a hydrophobic naturally or
   non-naturally occurring amino acid
<221> VARIANT
   <222> 23
   <223> Xaa = Absent or Gly or hydrophobic or small non-naturally
   occurring amino acid or replaced by an amide
<221> VARIANT
   <222> 24
   <223> Xaa = Absent or Arg or a basic naturally or non-naturally
   occurring amino acid
<221> VARIANT
   <222> 25
   <223> Xaa = Absent or Arg or a basic naturally or non-naturally
   occurring amino acid
<221> VARIANT
   <222> 26
   <223> Xaa = Ala or a hydrophobic or small naturally or
   non-naturally occurring amino acid
<221> VARIANT
   <222> 27
   <223> Xaa = Tyr or a hydrophobic or an aromatic naturally or
   non-naturally occurring amino acid
<221> VARIANT
   <222> 28
   <223> Xaa = Ala or a hydrophobic or small naturally or
   non-naturally occurring amino acid
<221> VARIANT
   <222> 29
   <223> Xaa = Ala or a hydrophobic or small naturally or
   non-naturally occurring amino acid
<221> VARIANT
   <222> 30
   <223> Xaa = Phe or a hydrophobic naturally or non-naturally
   occurring amino acid
<221> VARIANT
   <222> 31
   <223> Xaa = Ser or Thr or a polar naturally or non-naturally
   occurring amino acid
<221> VARIANT
   <222> 32
   <223> Xaa = Val or a hydrophobic naturally or non-naturally
   occurring amino acid
<221> VARIANT
   <222> 33
   <223> Xaa = Absent or Gly or hydrophobic or small naturally
   or non-naturally occurring amino acid or replaced by an amide
<400> 4
   Xaa Xaa Xaa Xaa Xaa Xaa Xaa Xaa Xaa Xaa Xaa Xaa Xaa Xaa Xaa Xaa
    1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa
<210> 5
   <211> 22
   <212> PRT
   <213> Homo sapiens
<400> 5
   Gly Gln Lys Gly Gln Val Gly Pro Pro Gly Ala Ala Val Arg Arg Ala
    1              5                   10                  15
   Tyr Ala Ala Phe Ser Val
               20
<210> 6
   <211> 32
   <212> PRT
   <213> Homo sapiens
<400> 6
   Gly Gln Lys Gly Gln Val Gly Pro Pro Gly Ala Ala Val Arg Arg Ala
    1              5                   10                  15
   Tyr Ala Ala Phe Ser Val Gly Arg Arg Ala Tyr Ala Ala Phe Ser Val
               20                  25                  30
<210> 7
   <211> 23
   <212> PRT
   <213> Homo sapiens
<400> 7
   Gly Gln Lys Gly Gln Val Gly Pro Pro Gly Ala Ala Cys Arg Arg Ala
    1              5                   10                  15
   Tyr Ala Ala Phe Ser Val Gly
               20
<210> 8
   <211> 262
   <212> PRT
   <213> Homo sapiens
<400> 8
   Met Ala Ala Pro Ala Leu Leu Leu Leu Ala Leu Leu Leu Pro Val Gly
    1               5                  10                  15
   Ala Trp Pro Gly Leu Pro Arg Arg Pro Cys Val His Cys Cys Arg Pro
               20                  25                  30
   Ala Trp Pro Pro Gly Pro Tyr Ala Arg Val Ser Asp Arg Asp Leu Trp
           35                  40                  45
   Arg Gly Asp Leu Trp Arg Gly Leu Pro Arg Val Arg Pro Thr Ile Asp
       50                  55                  60
   Ile Glu Ile Leu Lys Gly Glu Lys Gly Glu Ala Gly Val Arg Gly Arg
   65                  70                  75                  80
   Ala Gly Arg Ser Gly Lys Glu Gly Pro Pro Gly Ala Arg Gly Leu Gln
                   85                  90                  95
   Gly Arg Arg Gly Gln Lys Gly Gln Val Gly Pro Pro Gly Ala Ala Cys
               100                 105                 110
   Arg Arg Ala Tyr Ala Ala Phe Ser Val Gly Arg Arg Ala Tyr Ala Ala
           115                 120                 125
   Phe Ser Val Gly Arg Arg Glu Gly Leu His Ser Ser Asp His Phe Gln
       130                 135                 140
   Ala Val Pro Phe Asp Thr Glu Leu Val Asn Leu Asp Gly Ala Phe Asp
   145                 150                 155                 160
   Leu Ala Ala Gly Arg Phe Leu Cys Thr Val Pro Gly Val Tyr Phe Leu
                   165                 170                 175
   Ser Leu Asn Val His Thr Trp Asn Tyr Lys Glu Thr Tyr Leu His Ile
               180                 185                 190
   Met Leu Asn Arg Arg Pro Ala Ala Val Leu Tyr Ala Gln Pro Ser Glu
           195                 200                 205
   Arg Ser Val Met Gln Ala Gln Ser Leu Met Leu Leu Leu Ala Ala Gly
       210                 215                 220
   Asp Ala Val Trp Val Arg Met Phe Gln Arg Asp Arg Asp Asn Ala Ile
   225                 230                 235                 240
   Tyr Gly Glu His Gly Asp Leu Tyr Ile Thr Phe Ser Gly His Leu Val
                   245                 250                 255
   Lys Pro Ala Ala Glu Leu
               260

## Description

This application claims benefit under 35 U.S.C. 119(e) of U.S. Provisional Patent Application No. 61/127,888, filed May 16, 2008.

### FIELD

The present application is described in claims 1-7.

### BACKGROUND

Relaxin is a disulfide bridged polypeptide hormone of approximately 6000 daltons, which shows a marked increase in concentration during pregnancy in many species. Several studies have indicated that relaxin plays an important role during pregnancy and parturition by remodeling the reproductive tract.

Relaxin is mainly produced by the corpus luteum, in both pregnant and non-pregnant females and is also found in the seminal fluid of males (Bryant-Greenwood (1982), Endocrine Reviews 3, 62-90; Weisse (1984), Ann. Rev. Physiol. 46, 43-52). In females, its levels peak in peripheral plasma 7 to 10 days after the midcycle surge of luteinizing hormone and continue to rise if conception has occurred with additional relaxin being produced by the decidua (Stewart et al. (1990), J. Clin. Endo. Metab. 70, 1771-1773). During pregnancy serum concentrations of relaxin as measured by a homologous radioimmunoassay are highest by about the tenth week, and subsequently taper off and remain at about 500 pg/ml for the remainder of pregnancy (Bell et al. (1987), Obstet. Gynecol. 69, 585-589). Human gene forms for relaxin have been identified by genomic cloning using probes from the porcine relaxin gene (Hudson et al. (1983), Nature 301, 628; Hudson et al. (1984), EMBO J. 3, 2333; and U.S. Patent Nos. 4,758,516 and 4,871,670). Only one of these gene forms, termed H2, has been found to be transcribed and expressed in the corpora lutea in the ovary, suggesting that this is the sequence that is directly involved in the physiology of pregnancy.

In animals, relaxin has been described to remodel the reproductive tract in order o facilitate the birth process, including ripening of the cervix, thickening of the endometrium of the pregnant uterus, as well as increasing vascularization to this area and modifying collagen synthesis (Sherwood (1994) The physiology of reproduction 2.nd ed.). It has also been associated with lactation, and some reports indicate that relaxin has a growth-promoting effect on mammary tissue (Wright and Anderson (1982), Adv. Exp. Med. Biol. 143, 341).

The role of human relaxin during pregnancy is still poorly understood. *In vitro* studies of the effect of human relaxin suggest that it inhibits myometrial contractions of nonpregnant uteri, but does not affect the myometrium of pregnancy (MacLennan (1991), J. Reprod. Med. 36, 630-34). To date, no stimulatory effect of human relaxin on the myometrium has been reported. Notably, porcine relaxin has been shown to have an effect in humans, indicating that relaxin function is species specific. Evans *et al.,* utilizing porcine relaxin as an intracervical pellet in a randomized, double-blind, controlled clinical trial, observed greater cervical effacement (thinning) and a shorter time to delivery in the relaxin group (Evans et al. (1983), Am J. Obstet. Gynecol. 147, 410-414). Using a similar study design, *MacLennan et al.* administered porcine relaxin or placebo as an intracervical gel (MacLennan (1986), Obstet. Gynecol. 68, 595-601). The relaxin treated group was more likely to go into spontaneous labor, and there was a greater change in cervical ripening with an increased dose of relaxin. These studies suggest that porcine relaxin may be useful in labor induction in women. Porcine relaxin, however, may be immunogenic in women.

Human relaxin, administered intravaginally, has been tested in phase 1 safety studies in nonpregnant women, but did not show any effect on cervical ripening. This study demonstrated that administration of human relaxin is safe and associated with minimal adverse side effects. Importantly, serum levels of relaxin did not increase in females treated with relaxin, suggesting that the lack of an effect of relaxin on the cervix may be explained by inadequate or inappropriate method of dosing (Bell et al. (1993), Obstet. Gynecol. 82: 328-333; Brennand et al. (1995) 2nd International congress on the hormone relaxin, 380-388). Based on the function of relaxin in animals and the observations that porcine relaxin is able to promote cervical ripening in humans, it has been suggested that administration of relaxin can be used to facilitate parturition in humans.

Most pregnancies last about 40 weeks of gestation counted from the last menstrual period (LMP). Babies born at a gestational age of about 37 to 42 weeks are considered full term. About 12.5 percent of babies (more than half a million a year) in the United States are born prematurely before 37 completed weeks of pregnancy. Premature birth is a serious health problem and accounts for approximately 85% of morbidity and mortality in newborns in the United States. It represents the major part of neonatal health care costs, largely due to the 2% of American women delivering very premature infants who are born before 32 completed weeks of pregnancy.

Premature babies are born before their bodies and organ systems have completely developed. These babies are often small, with low birth weight (less than 2,500 grams or 5.5 pounds), and they may need help breathing, eating, fighting infection, and staying warm. They are at increased risk for newborn health complications, as well as lasting disabilities, such as mental retardation, cerebral palsy, lung and gastrointestinal problems, vision and hearing loss, and even death. Common complications that are more likely in premature than full-term babies include, but are not limited to, respiratory distress syndrome (RDS), apnea, intraventricular hemorrhage (IVH), patent ductus arteriosis, necrotizing enterocolitis (NEC), retinopathy of prematurity, jaundice, anemia, chronic lung disease and infections. Late preterm babies generally have few or mild problems, but babies born before about 32 to 34 weeks gestation may have a number of complications, ranging from mild to severe. Survival is possible for babies born as early as 24 to 27 weeks, but these babies may face a lifetime of health problems.

For reasons that are not fully understood, the rate of premature birth in the United States has increased by more than 30 percent in the last 25 years. Much of this increase may be accounted for by the increase in multiple gestations brought about by assisted reproductive technology. About 20% to 25% of all pregnancies conceived following such procedures result in twin gestation, which are at a particularly increased risk of preterm labor. Approximately 50% of twin gestations deliver prematurely, resulting in a mean gestational age of 37 weeks compared to 40 weeks for singleton pregnancies.

To date, there is no reliable and safe treatment to prevent premature delivery or preterm labor. Current methods include self-care methods to reduce infections, as well as nutritional and psychological interventions. Furthermore, these methods include intensive and frequent antenatal follow ups and the control of preterm birth risk factors. However, these methods have failed to demonstrate a significant reduction in preterm births. In case of premature uterine contractions, tocolytic therapy is usually applied. Various types of agents with varying success rates and side effects such as magnesium sulfate, nifedipine and terbutaline have been described. Some medications are not specifically Food and Drug Administration (FDA) approved for use in stopping uterine contractions in preterm labor, instead, they are being used off label. The suppression of contractions is often only partial and tocolytics can only be relied on to delay birth for several days. This method can be used to buy time for the administration of glucocorticoid drugs such as betamethasone, which greatly accelerate fetal lung maturity to reduce the risk of respiratory distress syndrome, intraventricular hemorrhage, necrotizing enterocolitis, infection and infant death. Glucocorticoid drugs take one to two days to work. Antibiotics have also been studied for their ability to prevent premature birth by fighting infections, but have not been proven effective. Furthermore, the use of vaginal progesterone, as well as injection with 17 alpha-hydroxyprogesterone caproate, a natural metabolite of progesterone, have been tested (Fonseca et al. (2007), NEJM, 357 (5), 462-469; Romero (2007), Ultrasound Obstet. Gynecol., 30, 675-686). This approach is based on the finding that the onset of labor is associated with a physiological withdrawal of progesterone. Both treatments were effective in stopping uterine contractions, however, the safety of 17 alpha-hydroxyprogesterone caproate has been recently questioned by the FDA and its expert panel due to an associated increase in miscarriage and fetal death *(*Meis et al. (2003), NEJM 348(24), 2379-85; and FDA advisory committees: CDER 2006)

Accordingly, there is a need for a safe and efficient method to prevent premature delivery. The disclosure described herein provides such a method and additional benefits as well.

### BRIEF SUMMARY OF THE PREFERRED EMBODIMENTS

The present application provides methods that are associated with reducing cervical dilation by administering relaxin as indicated in claims 1-7. Herein described are means which can be applied to decrease the risk of premature birth and miscarriage and to prolong the length of pregnancy, which can result in an increased birth weight of the infant. The number of premature deliveries has noticeably increased in U.S. hospitals over the past decade. Despite attempts to prevent such premature birth, no safe and efficient treatment without negative side effects has been found to date. Several methods have focused on self-care methods, which may help in some instances, but are not applicable in other cases. Thus, it is desirable to provide new means for preventing premature deliveries that are safe and effective and the disclosure meets this need. Herein described are means for preventing premature deliveries and decreasing the risk thereof by administering relaxin. One advantage is that administration of relaxin has little to no adverse side effects in women prone to premature delivery. Another advantage is that relaxin can be administered in combination with other medications, for example tocolytic agents or progesterone, to provide an even more potent method to prevent fetal prematurity. Since tocolytics and progesterone affect uterine contractions, whereas relaxin prevents cervical dilation, these treatments in combination provide a significant improvement compared to known methods.

Herein described are means of reducing cervical dilation including selecting a human female prone to premature delivery and administering a pharmaceutical formulation of pharmaceutically active relaxin to the female. Herein described are means of reducing rate of cervical dilation comprising, administering a pharmaceutical formulation comprising pharmaceutically active relaxin to a pregnant human female to reduce the rate of cervical dilation. Herein described are means of decreasing the risk of premature birth including selecting a human female prone to premature birth and administering a pharmaceutical formulation comprising pharmaceutically active relaxin to the female. Herein described are means of decreasing risk of premature birth, comprising administering a pharmaceutical formulation comprising pharmaceutically active relaxin to a pregnant human female to decrease the risk of premature birth. Herein described are means of reducing the risk of a miscarriage by selecting a human female prone to miscarriage and administering a pharmaceutical formulation comprising pharmaceutically active relaxin to the female. In the present uses the human female is a pregnant female as claimed. Pelaxin can be administered to a female prone to premature delivery before pregnancy. Notably, as described herein, relaxin can be administered to a pregnant female when she is experiencing symptoms including, but not limited to, contractions, cramps in her lower abdomen, low dull pain in her back, pressure in her pelvic area, stomach cramps, vaginal discharge, vaginal bleeding and vaginal watery fluid leakage.

Herein described are means of increasing the length of a term of pregnancy including selecting a pregnant human female prone to premature delivery and administering a pharmaceutical formulation of pharmaceutically active relaxin, which reduces the risk of premature delivery. Herein described is the increase of infant birth weight comprising selecting a pregnant human female prone to premature delivery, and administering a pharmaceutical formulation of pharmaceutically active relaxin to said female to increase birth rate by increasing length of pregnancy. Relaxin can administered to a pregnant female when she is experiencing symptoms including, but not limited to, contractions, cramps in her lower abdomen, low dull pain in her back, pressure in her pelvic area, stomach cramps, vaginal discharge, vaginal bleeding and vaginal watery fluid leakage.

Relaxin employed in the pharmaceutical formulations of the disclosure can be, for example, purified, synthetic or recombinant relaxin. The relaxin is H2 human relaxin. H1 human relaxin, H3 human relaxin or a relaxin agonist are herein described. In another embodiment, relaxin is synthetic or recombinant H2 human relaxin. Thus, the subject can be treated with a pharmaceutical formulation of synthetic or recombinant human relaxin. In one embodiment of the disclosure, the subject is treated with synthetic H2 human relaxin. In yet another embodiment, the subject is treated with recombinant H2 human relaxin. Relaxin can be administered to the subject through a number of different routes, including but not limited to, intravenously, subcutaneously, intramuscularly, or topically. In one preferred embodiment, relaxin is administered intravenously. In another preferred embodiment, relaxin is administered subcutaneously. In yet a further embodiment relaxin is administered through spinal injection. More specifically, the pharmaceutical formulation of relaxin can be administered to the subject in an amount in a range of about 0.1 to 500 µg/kg of subject body weight per day. As such, relaxin is administered to the subject so as to maintain a serum concentration of relaxin of from about 0.5 to 50 ng/ml.

Administration of relaxin prevents premature labor or premature birth in a female at risk of these indications. Furthermore, administration of relaxin can lead to increased infant weight at birth and/or to normal infant weight at birth. In one embodiment of the methods of the disclosure, H2 relaxin is administered from the beginning of the first trimester of pregnancy until the onset of labor. In another embodiment, relaxin H2 is administered from the beginning of the second or third trimester until the onset of labor. H2 relaxin can be administered daily, weekly or monthly during these periods.

Herein described is relaxin for use in reducing rate of cervical dilation in a human female prone to premature delivery; relaxin for use in decreasing risk of premature delivery and miscarriage in a human female prone to premature delivery or miscarriage; and relaxin for use in extending the length of term of pregnancy and increasing infant birth weight in a human female prone to premature delivery.

### BRIEF DESCRIPTION OF THE DRAWINGS

Figure 1A depicts the peptide hormone H2 relaxin which is similar in size and shape to insulin. Figure 1B provides the amino acid sequence of the B chain (SEQ ID NO: 1) and the A chain (SEQ ID NO:2 with X representing glutamic acid [E] or glutamine [Q]) of human relaxin 2 (H2).
Figure 2 shows the mean serum relaxin concentrations in the pooled relaxin and pooled placebo groups over time.
Figure 3 shows the mean total Bishop scores in the pooled relaxin and pooled placebo groups at the indicated time points.
Figure 4 shows the change in cervical dilation in the pooled relaxin and pooled placebo groups over time.

### DETAILED DESCRIPTION

### General Overview

The present disclosure is based on the observation that intravenous administration of human relaxin to pregnant females results in a significant reduction in rate of cervical dilation. Cervical dilation denotes an important parameter in determining the physical readiness of woman to progress toward vaginal delivery. Surprisingly, the inventor has found that administration of relaxin can be applied to inhibit cervical dilation in human females. This finding sharply contrasts with results obtained in animal studies, where administration of relaxin enhances cervical ripening and dilation. Notably, prematurely increased dilation increases the risk of preterm delivery. Thus, inhibition of cervical dilation provides means to preclude premature delivery before reaching the full term of pregnancy, as well as means to prevent miscarriages during the early stages of pregnancy. By preventing premature delivery, the length of pregnancy can be extended to ensure full development of the infant or at least to a maturity level of the infant's organs, which is sufficient for the infant's viability outside of the womb.

Premature birth poses health risks to both the baby and the mother, and is a costly health problem. Premature babies require special care, often suffer from serious diseases and may experience life-long health effects. Apart from these physical constrains, premature birth may also have a strong psychological impact on both infant and mother. Although a number of technological advances in perinatal and neonatal medicine have been made to increase the chances of survival and recovery from medical complications of premature babies, premature birth remains one of the top causes for death in infants within their first year of life.

### Definitions

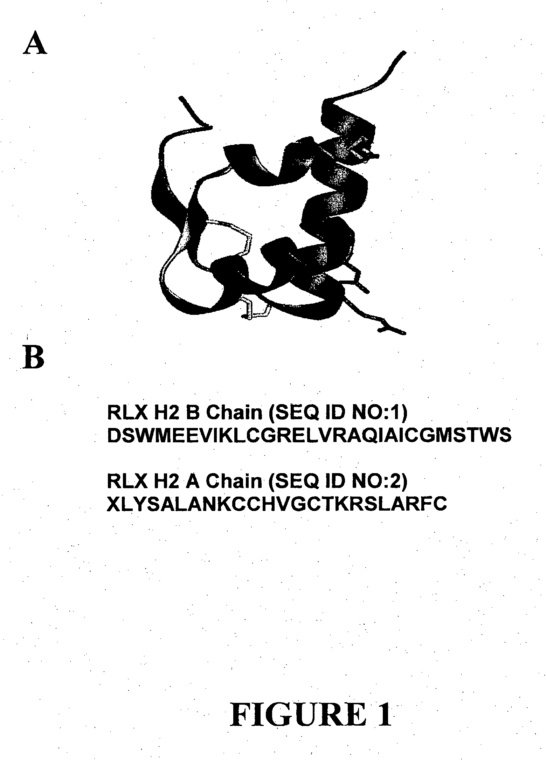

The term "relaxin" refers to a peptide hormone which is well known in the art (see Figure 1). The term "relaxin", as used herein, encompasses human relaxin, including intact full length human relaxin or a portion of the relaxin molecule that retains biological activity. The term "relaxin" further contemplates synthetic human relaxin and recombinant human relaxin, including synthetic H2 human relaxin and recombinant H2 human relaxin. The term further encompasses active agents with relaxin-like activity, such as relaxin analogs and portions thereof that retain biological activity, and agents that competitively displace bound relaxin from a relaxin receptor such as an LGR7 or an LGR8 receptor. In addition, the nucleic acid sequence of human relaxin as used herein must not be 100% identical to human relaxin H2 but may be at least about 40%, 50%, 60%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to human relaxin H2. Relaxin, as used herein, can be made by any method known to those skilled in the art. Examples of such methods are illustrated, for example, in U.S. Patent No. 5,759,807 as well as in Bullesbach et al. (1991) The Journal of Biological Chemistry 266(17):10754-10761. Examples of relaxin molecules and analogs are illustrated, for example, in U.S. Patent No. 5,166,191.

The term "cervical dilation" refers to the process in which the cervical os (opening) and the cervical canal widen from less than about a centimeter to approximately about 10 cm, allowing delivery of the fetus.

The term "cervical ripening" refers to the process that prepares the cervix for labor, for example making the cervix soft and thin *(i.e.,* effacement).

The terms "premature delivery", "premature birth", 'preterm birth" and "preterm delivery" refer to birth before around 37 completed weeks of pregnancy and are used interchangeably throughout the specification.

The terms "premature labor" or "preterm labor" refer to the onset of labor before around 37 completed weeks of pregnancy and are characterized by the presence of uterine contractions or increased uterine irritability. The terms "premature labor" or "preterm labor" are used interchangeably throughout the specification.

The term "miscarriage" or "spontaneous abortion" refer to the natural or spontaneous end of a pregnancy at a stage where the embryo or the fetus is incapable of surviving, generally defined in humans at a gestation of prior to about 20 weeks. These terms are used interchangeably throughout the specification.

The term "infant" or "baby" refers to a human child at the youngest stage of life, specifically before they can walk and generally before the age of one These terms are used interchangeably throughout the specification.

The term "newborn infant" or "neonate" refers to a human infant less than about a month old. These terms are used interchangeably throughout the specification and include premature, post mature and full term infants.

The term "about" when used in the context of a stated value, encompasses a range of up to 10% above or below the stated value (e.g., 90-110% of the stated value). For instance, an intravenous (IV) infusion rate of about 30 mcg/kg/day, encompasses IV infusion rates of 27 mcg/kg/day to 33 mcg/kg/day.

"Therapeutically effective" refers to the amount of pharmaceutically active relaxin that will result in a measurable desired medical or clinical benefit to a patient, as compared to the patient's baseline status or to the status of an untreated or placebo-treated (e.g., not treated with relaxin) subject.

### Relaxin

Relaxin is a peptide hormone that is similar in size and shape to insulin (see Figure 1). More specifically, relaxin is an endocrine and autocrine/paracrine hormone which belongs to the insulin gene superfamily. The active form of the encoded protein consists of an A chain and a B chain, held together by disulphide bonds, two inter-chains and one intra-chain. Thus, the structure closely resembles insulin in the disposition of disulphide bonds. In humans, there are three non-allelic relaxin genes, relaxin-1 (RLN-1 or H1), relaxin-2 (RLN-2 or H2) and relaxin-3 (RLN-3 or H3). H1 and H2 share high sequence homology. There are two alternatively spliced transcript variants encoding different isoforms described for this gene. Expression of H1 in humans is uncertain. H2 is expressed in reproductive organs while H3 is found primarily in the brain. The evolution of the relaxin peptide family in its receptors is generally well known in the art (see Wilkinson et al. (2005) BMC Evolutionary Biology 5(14):1-17; and Wilkinson and Bathgate (2007) Chapter 1, Relaxin and Related Peptides, Landes Bioscience and Springer Science + Business Media*).*

Relaxin activates two specific relaxin receptors, i.e., LGR7 (RXFP1) and LGR8 (RXFP2). LGR7 and LGR8 are leucine-rich repeat-containing, G protein-coupled receptors (LGRs) which represent a unique subgroup of G protein-coupled receptors. They contain a heptahelical transmembrane domain and a large glycosylated ectodomain, distantly related to the receptors for the glycoproteohormones, such as the LH-receptor or FSH-receptor. These relaxin receptors are found in the heart, smooth muscle, connective tissue, and central and autonomous nervous system. Potent relaxins such as H1, H2, porcine and whale relaxin possess a certain sequence in common, *i.e.,* the Arg-Glu-Leu-Val-Arg-X-X-Ile (SEQ ID NO:3) sequence or binding cassette. Relaxins that deviate from his sequence homology such as rat, shark, dog and horse relaxins show a reduction in bioactivity through the LGR7 and LGR8 receptors (see Bathgate et al. (2005) Ann. N.Y. Acad. Sci. 1041:61-76; Receptors for Relaxin Family Peptides*).*

Relaxin is found in both, women and men (see Tregear et al.; Relaxin 2000, Proceedings of the Third International Conference on Relaxin & Related Peptides (22-27 October 2000, Broome, Australia). In women, relaxin is produced by the *corpus luteum* of the ovary, the breast and, during pregnancy, also by the placenta, chorion, and decidua. In men, relaxin is produced in the testes. Relaxin levels rise after ovulation as a result of its production by the *corpus luteum* and its peak is reached during the first trimester, not toward the end of pregnancy. In the absence of pregnancy its level declines. In humans, relaxin is plays a role in pregnancy, in enhancing sperm motility, regulating blood pressure, controlling heart rate and releasing oxytocin and vasopressin. In animals, relaxin widens the pubic bone, facilitates labor, softens the cervix (cervical ripening), and relaxes the uterine musculature. In animals, relaxin also affects collagen metabolism, inhibiting collagen synthesis and enhancing its breakdown by increasing matrix metalloproteinases. It also enhances angiogenesis and is a renal vasodilator.

Relaxin has the general properties of a growth factor and is capable of altering the nature of connective tissue and influencing smooth muscle contraction. H2 is known to be primarily expressed in reproductive tissue (see U.S. Patent No. 5,023,321) and has been implicated in cervical dilation in animals. However, the inventor has discovered that in humans, relaxin causes the opposite effect and in fact reduces the rate of cervical dilation.

### Relaxin Agonists

Herein described is the administration of a relaxin agonist. The relaxin agonist activates one or more relaxin-related G-protein coupled receptors (GPCR) selected from but not limited to RXFP1, RXFP2, RXFP3, RXFP4, FSHR (LGR1), LHCGR (LGR2), TSHR (LGR3), LGR4, LGR5, LGR6 LGR7 (RXFP1) and LGR8 (RXFP2). The relaxin agonist comprises the amino acid sequence of Formula I of WO 2009/007848 of Compugen.

Formula I peptides are preferably from 7 to 100 amino acids in length and comprise the amino acid sequence: X1- X2- X3- X4- X5- X6- X7- X8- X9- X10- X1 1- X12- X13- X14-X15- X16- X17- X18- X19- X20-X21- X22- X23- X24- X25- X26- X27- X28- X29- X30- X31-X32- X33; wherein X1 is absent or G or a small naturally or non-naturally occurring amino acid; X2 is absent or Q or a polar naturally or non-naturally occurring amino acid; X3 is absent or K or a basic naturally or non-naturally occurring amino acid; X4 is absent or G or a small naturally or non-naturally occurring amino acid; X5 is absent or Q or S a polar naturally or non-naturally occurring amino acid; X6 is absent or V or A or P or M or a hydrophobic naturally or non-naturally occurring amino acid; X7 is absent or G or a small naturally or non-naturally occurring amino acid; X8 is absent or P or L or A naturally or non-naturally occurring amino acid; X9 is absent or P or Q naturally or non-naturally occurring amino acid; X10 is absent or G or a small naturally or non-naturally occurring amino acid; X11 is absent or A or H or E or D or a hydrophobic or a small or an acidic naturally or non-naturally occurring amino acid; X12 is absent or A or P or Q or S or R or H or a hydrophobic or a small naturally or non- naturally occurring amino acid; X13 is absent or C or V or a hydrophobic naturally or non-naturally occurring amino acid; X14 is absent or R or K or Q or P or a basic or a polar naturally or non-naturally occurring amino acid; X15 is absent or R or Q or S or a basic or a polar naturally or non-naturally occurring amino acid; X16 is absent or A or L or H or Q or a hydrophobic or a small naturally or non-naturally occurring amino acid; X17 is absent or Y or a hydrophobic or an aromatic naturally or non-naturally occurring amino acid; X18 is absent or A or a hydrophobic or small naturally or non-naturally occurring amino acid; X19 is absent or A or a hydrophobic small naturally or non-naturally occurring amino acid; X20 is absent or F or a hydrophobic or an aromatic naturally or non-naturally occurring amino acid; X21 is absent or S or T or a polar naturally or non-naturally occurring amino acid; X22 is absent or V or a hydrophobic naturally or non-naturally occurring amino acid; X23 is absent or G or hydrophobic or small non-naturally occurring amino acid or replaced by an amide; X24 is absent or R or a basic naturally or non-naturally occurring amino acid; X25 is absent or R or a basic naturally or non-naturally occurring amino acid; X26 is A or a hydrophobic or small naturally or non-naturally occurring amino acid; X27 is Y or a hydrophobic or an aromatic naturally or non-naturally occurring amino acid; X28 is A or a hydrophobic or small naturally or non-naturally occurring amino acid; X29 is A or a hydrophobic or small naturally or non-naturally occurring amino acid; X30 is F or a hydrophobic naturally or non-naturally occurring amino acid; X31 is S or T or a polar naturally or non-naturally occurring amino acid; X32 is V or a hydrophobic naturally or non-naturally occurring amino acid; X33 is absent or G or hydrophobic or small naturally or non-naturally occurring amino acid or replaced by an amide; or a pharmaceutically acceptable salt thereof (SEQ ID NO:4). The relaxin agonist comprises the sequence of peptide P59C13V (free acid) GQKGQVGPPGAA VRRA Y AAFSV (SEQ ID NO:5). In another preferred embodiment, the relaxin agonist comprises the sequence of peptide P74C13V (free acid) GQKGQVGPPGAA VRRA Y AAFS VGRRA Y AAFS V (SEQ DD NO: 6). Further derivatives of the human complement C1Q tumor necrosis factor-related protein 8 (CTRP8 or C1QT8) such as peptide P59-G (free acid Gly) GQKGQVGPPGAACRRA Y AAFSVG (SEQ ID NO:7) are also contemplated to be suitable for use in the methods of the present disclosure. The amino acid sequence of C1QT8 is set forth as SEQ ID NO:8 MAAPALLLLALLLPVGAWPGLPRRPCVHCCRPAWPPGPYARVSDRDLWRGDLWRGLP RVRPTIDIEILKGEKGEAGVRGRAGRSGKEGPPGARGLQGRRGQKGQVGPPGAACRRA YAAFSVGRRAYAAFSVGRREGLHSSDHFQAVPFDTELVNLDGAFDLAAGRFLCTVPGV YFLSLNVHTWNYKETYLHIMLNRRPAAVLYAQPSERSVMQAQSLMLLLAAGDAVWVR MF QRDRDNAIYGEHGDLYITFSGHLVKP AAEL.

Herein described are homologues of these polypeptides, such homologues can be at least 50%, at least 55%, at least 60%, at least 65%, at least 70%, at least 75%, at least 80%, at least 85%, at least 85%, at least 90%, at least 95% or more say 100% identical to the amino acid sequence of an exemplary relaxin agonist (e.g., SEQ ID NO:5 or SEQ ID NO:6), as can be determined using BlastP software of the National Center of Biotechnology Information (NCBI) using default parameters, optionally and preferably including the following: filtering on (this option filters repetitive or low- complexity sequences from the query using the Seg (protein) program), scoring matrix is BLOSUM62 for proteins, word size is 3, E value is 10, gap costs are 1 1, 1 (initialization and (initialization and extension). Optionally and preferably, nucleic acid sequence identity/homology is determined with BlastN software of the National Center of Biotechnology Information (NCBI) using default parameters, which preferably include using the DUST filter program, and also preferably include having an E value of 10, filtering low complexity sequences and a word size of 1 1. Finally the present disclosure also encompasses fragments of the above described polypeptides and polypeptides having mutations, such as deletions, insertions or substitutions of one or more amino acids, either naturally occurring or artificially induced, either randomly or in a targeted fashion.

### Relaxin Treatment Results in Reduction of Rate of Cervical Dilation

Pregnancy is maintained by the absence of forceful uterine contractions and a closed, competent cervix. The cervix serves as a protective barrier from invading microorganism and as a structural barrier to delivery of the fetus. Failure of cervical competency leads to preterm birth even in the absence of uterine contractions of labor. However, forceful uterine contractions in the presence of a rigid cervix are insufficient to bring about delivery. Thus, timely changes in cervical function during gestation are crucial for successful pregnancy. During and after parturition the cervix undergoes a dramatic process of connective tissue remodeling, which occurs in four stages: softening, ripening, dilation and repair (Word et al., 2007 Semin. Reprod. Med. 25 (1):69-79). The initial softening phase is a slow, progressive process characterized by an increase in collagen synthesis and cervical growth and results in reorganization of the collagen fibrillar network. This process provides tensile strength of the softened cervix during the early stages of gestation. During cervical ripening, collagen concentrations are decreased primarily due to an increase in synthesis of hydrophilic glycosaminoglycans. At this stage, tensile strength is decreased and the cervix starts to efface (thin) and dilate. Dilation during labor is different from this initial dilation process during ripening and is caused by a different mechanism, which involves the release of proteases and collagenases into the extracellular matrix by leukocytes. The final phase of cervical remodeling, repair, occurs after parturition.

Notably, cervical ripening is necessary for cervical dilation and it is well established that both processes are required for successful parturition at term. The standard measurement for cervical ripening is the Bishop score. It represents a rating of how soft, open, and thinned the cervix is (dilation and effacement), as well as how low in the pelvis the cervix and baby are positioned, thereby assessing the readiness for vaginal delivery. Cervical ripening usually starts several weeks before onset of uterine contractions, suggesting that both preterm and term parturition in women is a process of long duration and that uterine contractions of labor are late events in the parturition process (Word *et al., supra*)*.* Relaxin has been implicated in facilitating the birth process by promoting cervical ripening and dilation in animals. Surprisingly, the inventor has found that administration of relaxin has the opposite effect in human females, and provides a method to reduce the rate of cervical dilation. Based on this finding, relaxin (for example, human relaxin or human H2 relaxin) can be administered to a pregnant female prone to premature delivery to reduce the rate of cervical dilation. Relaxin can be administered by several routes of administration including but not limited to intravenous, subcutaneous, intramuscular or topic administration or by spinal injection. Treatment with relaxin can start as early as the first trimester until the onset of labor, or alternatively, treatment can start at the beginning of the second or third trimester until the onset of labor.

### Relaxin Treatment Provides a Method to Prevent Premature Birth or Miscarriage

Current methods to prevent premature delivery are directed towards strategies and treatments to preclude premature labor, which is characterized by uterine contractions or increased uterine irritability before term. The actual physiologic mechanism that initiates preterm labor has not been clearly identified but has been shown to be influenced by such factors as prostaglandin synthesis, oxytocin release, hormonal ratios (decline in progesterone level, rise in estradiol level), mechanical stretch of the uterine tissues, and changes in uterine blood flow. In most cases, preterm labor results in premature birth, since no highly efficient and reliable method to stop labor exists to date. Therefore, reducing the incidence of preterm labor is thought to be crucial in order to reduce the risk of premature delivery. Accordingly, strategies have focused on educating both physicians and patients about the risks for preterm labor and methods of its early detection. These involve paying attention to typical symptoms of preterm labor, such as contractions that occur more than six times each hour, low, dull backache, a sensation of pelvic pressure, diarrhea, vaginal spotting or bleeding or watery vaginal discharge. In addition, weekly cervical assessment, transvaginal ultrasonography, detection of fetal fibronectin (as an indicator of disruption of the placental tissues) and home uterine activity monitoring are suggested in women with a high risk for premature labor and delivery (Weissmiller (1999), American Academy of Family Physicians*).* At present, management of preterm labor includes bed rest, reduced physical activity, and the use of tocolytic agents to prevent contractions, which do help in some cases, but are not sufficient in others.

The present invention as defined in claims 1-7 reduce the risk of preterm birth by reducing rate of premature cervical dilation rather than affecting uterine contractions. Notably, cervical ripening and dilation are thought to precede uterine contractions at term, as well as before term during premature labor (Word *et al., supra).* Thus, administration of relaxin is particularly suitable in cases where it is important to prevent cervical dilation. In other instances, uterine contractions during preterm labor cause dilation of the cervix. Administration of relaxin can be applied here as well to reduce rate of dilation, thereby preventing birth. Herein, relaxin can be used alone or in combination with agents that affect contractility (e.g., tocolytics or progesterone), providing an even more potent method to preclude premature birth.

Notably, ovarian hyper-stimulation or *in vitro* fertilization result in pregnancies with enhanced circulating relaxin levels when compared to normal pregnancies due to the presence of increased luteal tissue. Since these pregnancies were associated with a higher incidence of premature delivery, it was suggested that elevated serum levels of relaxin are linked to an early onset of labor (Weiss et al. (1993) Obstet Gynecol 82, 821-828). However, multiple factors in these pregnancies may play a role in increasing the risk of fetal prematurity. Importantly, administration of relaxin as described herein, results in several fold higher serum levels of relaxin compared to the levels observed in these pregnancies. The inventor shows that at these significantly higher levels, relaxin reduces cervical dilation, a prerequisite for vaginal delivery, and thus prevents premature delivery.

Herein described are means that reduce the risk of miscarriage. Miscarriage, the loss of a pregnancy without obvious cause before about the 20th week of pregnancy, is the most common complication of early pregnancy. About 15 percent of known pregnancies end in miscarriage, according to the American College of Obstetricians and Gynecologists (ACOG). But the actual number is probably much higher because many miscarriages occur so early in pregnancy that a woman is not even aware of being pregnant. Most miscarriages occur before the 12th week of pregnancy, sometimes due to genetic problems of the embryo, which are usually unrelated to the mother. Another cause of early spontaneous abortion may be progesterone deficiency. Progesterone supplements have been prescribed in these cases, but no study has shown that they are able to reduce the risk of miscarriage. Other causes of miscarriage, particularly during later stages of pregnancy, include, but are not limited to uterine malformations, growths in the uterus, cervical problems as well as problems with the umbilical cord or with the placenta. Although in many cases miscarriage is associated with fetal abnormalities, the loss of the product of conception before about week 20 of gestation is a disorder of unknown etiology.

Currently, a high risk of miscarriage in pregnant women is treated with bed rest. Miscarriages often present with abdominal cramping or vaginal bleeding. Other symptoms include, but are not limited to, low back pain or abdominal pain that is dull or sharp, vaginal spotting with or without abdominal cramps, fluid, tissue or clot-like material that passes from the vagina, fever and emesis. Strategies to reduce the risk of miscarriage focus mainly on reduced physical activity and bed rest, which is often debilitating to the expecting mother. In addition, no reliable method to prevent miscarriages existed prior to relaxin treatment as described herein. However, the present disclosure provides a reliable method to prevent miscarriage and reduce the risk of miscarriage through administration of relaxin. Miscarriage is often accompanied with cervical changes such as cervical dilation. Thus, administration of relaxin is highly applicable in these cases since it significantly reduces the rate of cervical dilation as demonstrated herein.

As a result of prevention of preterm delivery or miscarriage by administering relaxin, the length of term of pregnancy can be increased. In some instances, administration of relaxin can extend the pregnancy to full term, which is preferable as full term babies are at lower risk of many neonatal complications. Due to medical conditions of the mother or the baby, such as pre-eclampsia, vaginal bleeding or fetal distress, delivery before full term is sometimes required. In these cases relaxin can be administered to extend pregnancy to the stage where the infant's organs are sufficiently developed to ensure infant viability.

An important factor for the chances of survival of a newborn is birth weight. The average birth weight of a full-term newborn is approximately about 7.5 pounds (3.2 kg), but is typically in the range of about 5.5-10 pounds (2.7-4.6 kg). Birth weight in premature infants is categorized as follows: birth weight below 5 lb 8 oz (2500 g) is defined as low birth weight (LBW); weight below 3 lb 5 oz (1500 g) is very low birth weight (VLBW); and weight below 2 lb 3 oz (1000 g) is extremely low birth weight (ELBW). LBW, VLBW and ELBW are associated with higher risk for a variety of problems in the infant including, but not limited to, cerebral palsy, sepsis, chronic lung disease and death. These infants are also at higher risk for hypothermia, which can be dangerous. Birth weight below average is often observed in premature newborns. Thus, prevention of premature delivery using relaxin provides a means for increasing the birth weight of newborns. Notably, birth weight below average is sometimes also observed in full term babies. In certain cases, administration of relaxin can be used to extend pregnancy over full term in order to increase birth weight and chances of survival.

Herein described are means to reduce the risk of premature delivery and miscarriage, as well as means to increase the term of pregnancy and infant birth weight by administering relaxin. Relaxin (e.g., human H2 relaxin) can be administered to a female prone to premature delivery by several routes of administration including, but not limited to, intravenous, subcutaneous, intramuscular, topical administration or by spinal injection. Treatment with relaxin can start as early as the first trimester until the onset of labor, or alternatively, treatment can start at the beginning of the second or third trimester until the onset of labor.

### Females at Risk for Premature Delivery or Miscarriage

Women who have a history of preterm labor, premature delivery or miscarriages, as well as women pregnant with twins or multiples, or women pregnant with a single fetus after *in vitro* fertilization (IVF) are at higher risk for preterm birth. Furthermore, non-white women are at greater risk for preterm delivery as well as females younger than 18 or older than 35 years of age. Malnutrition, obesity, being underweight before pregnancy and being less than 5 feet of height are other factors increasing this risk. Another important aspect is the time between pregnancies, as short periods of 6 to 9 months between birth and the beginning of another pregnancy correlate with higher incidence of premature birth. Furthermore, women who have certain uterine or cervical abnormalities often deliver prematurely. For example, women with cervical incompetence, a condition in which the cervix begins to open (dilate) and thin (efface) without pain or uterine contractions before a pregnancy has reached term. These events occur because of a weakness in the cervix, which opens under the growing pressure of the uterus as pregnancy progresses. In addition, certain life style factors including, but not limited to, late or no prenatal care, smoking, alcohol consumption, use of illegal drugs, domestic violence, lack of social support, extremely high levels of stress and long working hours with long periods of standing, may also augment the risk for preterm birth. Certain medical conditions during pregnancy may also increase the likelihood that a woman will give birth before term. These include abnormal placentation, urinary tract infections, vaginal infections, sexually transmitted infections, and possibly, also other infections, diabetes, high blood pressure, clotting disorders (thrombophilia), bleeding from the vagina, certain birth defects in the baby and exposure to DES (diethylstilbestrol, a synthetic estrogen given to treat estrogen deficiency conditions).

Women at higher risk for miscarriage include females over 35 years of age or with a history of miscarriages and females with multiple gestations. Furthermore, hormonal insufficiency or imbalance, uterine or cervical abnormalities, infection with rubella, chlamydia or other sexually-transmitted infections, bacterial vaginosis, uncontrolled diabetes, thyroid problems, disorders of the immune system, severe kidney disease, and congenital heart disease augment the risk of miscarriage. In addition, lifestyle factors such as smoking, drinking alcohol or using illegal drugs increase the likelihood of spontaneous abortion, as well as certain medications, such as the acne drug accutane, severe malnutrition and exposure to environmental and workplace hazards such as high levels of radiation or toxic agents.

### Relaxin Compositions and Formulations

Relaxin and relaxin analogs are formulated as pharmaceuticals to be used in the methods of the disclosure. Any composition or compound that can stimulate a biological response associated with the binding of biologically or pharmaceutically active relaxin (e.g., synthetic relaxin, recombinant relaxin) or a relaxin agonist (e.g., relaxin analog or relaxin-like modulator) to relaxin receptors can be used as a pharmaceutical in the disclosure. General details on techniques for formulation and administration are well described in the scientific literature (see Remington's Pharmaceutical Sciences, Maack Publishing Co, Easton Pa.). Pharmaceutical formulations containing pharmaceutically active relaxin can be prepared according to any method known in the art for the manufacture of pharmaceuticals. The formulations containing pharmaceutically active relaxin or relaxin agonists used in the methods of the disclosure can be formulated for administration in any conventionally acceptable way including, but not limited to, intravenously, subcutaneously, intramuscularly, topically or through spinal injection. Illustrative examples are set forth below. In one preferred embodiment, relaxin is administered intravenously.

When the drugs are delivered by intravenous injection, the formulations containing pharmaceutically active relaxin or a relaxin agonist can be in the form of a sterile injectable preparation, such as a sterile injectable aqueous or oleaginous suspension. This suspension can be formulated according to the known art using those suitable dispersing or wetting agents and suspending agents which have been mentioned above. The sterile injectable preparation can also be a sterile injectable solution or suspension in a nontoxic parenterally-acceptable diluent or solvent. Among the acceptable vehicles and solvents that can be employed are water and Ringer's solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables.

Pharmaceutical formulations for topical administration contain a pharmaceutically acceptable topical carrier and dosages adequate for topical administration, and may be in any form suitable for application to the body surface. The formulations comprise, for example, a cream, lotion, solution, gel, ointment, paste, or the like. Various additives, known to those skilled in the art, can be included in the topical formulations. For example, solvents, including relatively small amounts of alcohol, can be used to solubilize certain formulation components. It can also be desirable to include an added permeation enhancer in the formulation. Permeation enhancers will be known to those of ordinary skill in the art of topical drug delivery, and/or are described in the pertinent texts and literature. See, e.g., Percutaneous Penetration Enhancers, eds. Smith et al. (CRC Press, 1995). The topical formulations can also include conventional additives such as opacifiers, antioxidants, fragrance, colorant, gelling agents, thickening agents, diluents, stabilizers, surfactants, and the like. Other agents can also be added, such as antimicrobial agents, to prevent spoilage upon storage, i.e., to inhibit growth of microbes such as yeasts and molds. The formulations can also contain irritation-mitigating additives to minimize or eliminate the possibility of skin irritation or skin damage resulting from the pharmacologically active ingredient or other components of the composition.

Pharmaceutical formulations for oral administration can be formulated using pharmaceutically acceptable carriers well known in the art in dosages suitable for oral administration. Such carriers enable the pharmaceutical formulations to be formulated in unit dosage forms as tablets, pills, powder, capsules, liquids, lozenges, gels, syrups, slurries, suspensions, *etc.,* suitable for ingestion by the patient. Pharmaceutical preparations for oral use can be obtained through combination of relaxin compounds with a solid excipient, optionally grinding a resulting mixture, and processing the mixture of granules, after adding suitable additional compounds, if desired, to obtain tablets or pills. Suitable solid excipients are carbohydrate or protein fillers which include, but are not limited to, sugars, including lactose, sucrose, mannitol, or sorbitol; starch from corn, wheat, rice, potato, or other plants; cellulose such as methyl cellulose, hydroxypropylmethyl-cellulose, or sodium carboxymethylcellulose; and gums including arabic and tragacanth; as well as proteins such as gelatin and collagen. If desired, disintegrating or solubilizing agents may be added, such as the cross-linked polyvinyl pyrrolidone, agar, alginic acid, or a salt thereof, such as sodium alginate. Pharmaceutical preparations of the disclosure that can also be used orally are, for example, push-fit capsules made of gelatin, as well as soft, sealed capsules made of gelatin and a coating such as glycerol or sorbitol. Push-fit capsules can contain relaxin mixed with a filler or binders such as lactose or starches, lubricants such as talc or magnesium stearate, and, optionally, stabilizers. In soft capsules, the relaxin compounds may be dissolved or suspended in suitable liquids, such as fatty oils, liquid paraffin, or liquid polyethylene glycol with or without stabilizers.

Aqueous suspensions herein described contain relaxin in admixture with excipients suitable for the manufacture of aqueous suspensions. Such excipients include a suspending agent, such as sodium carboxymethylcellulose, methylcellulose, hydroxypropylnethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (*e.g.,* polyoxyethylene stearate), a condensation product of ethylene oxide with a long chain aliphatic alcohol *(e.g.,* heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan monooleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

Oil suspensions can be formulated by suspending relaxin in a vegetable oil, such as arachis oil, olive oil, sesame oil or coconut oil, or in a mineral oil such as liquid paraffin. The oil suspensions can contain a thickening agent, such as beeswax, hard paraffin or cetyl alcohol. Sweetening agents can be added to provide a palatable oral preparation. These formulations can be preserved by the addition of an antioxidant such as ascorbic acid.

Dispersible powders and granules herein described suitable for preparation of an aqueous suspension by the addition of water can be formulated from relaxin in admixture with a dispersing, suspending and/or wetting agent, and one or more preservatives. Suitable dispersing or wetting agents and suspending agents are exemplified by those disclosed above. Additional excipients, for example sweetening, flavoring and coloring agents, can also be present.

The pharmaceutical formulations herein described can also be in the form of oil-in-water emulsions. The oily phase can be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening and flavoring agents. Syrups and elixirs can be formulated with sweetening agents, such as glycerol, sorbitol or sucrose. Such formulations can also contain a demulcent, a preservative, a flavoring or a coloring agent.

### Administration and Dosing Regimen

The formulations containing pharmaceutically active relaxin herein described can be administered in any conventionally acceptable way including, but not limited to, intravenously, subcutaneously, intramuscularly, topically, and through spinal injection. Administration will vary with the pharmacokinetics and other properties of the drugs and the patients' condition of health. General guidelines are presented below.

The invention as defined in claims 1-7 reduces cervical dilation in human females prone to premature delivery. Herein described are means to reduce the risk of premature delivery and miscarriage in females prone to premature delivery or miscarriage as well as to increase the length of term of pregnancy and increase infant birth weight in these subjects. The amount of relaxin alone or in combination with another agent or drug (e.g., progesterone or tocolytic agents) that is adequate to accomplish this is considered the therapeutically effective dose. The dosage schedule and amounts effective for this use, *i.e.,* the "dosing regimen," will depend upon a variety of factors, including the stage of pregnancy, the extent of preterm cervical dilation, the severity of preterm labor, the severity of the adverse side effects, the general state of the patient's health, the patient's physical status, age and the like. In calculating the dosage regimen for a patient, the mode of administration is also taken into consideration. The dosage regimen must also take into consideration the pharmacokinetics, *i.e.,* the rate of absorption, bioavailability, metabolism, clearance, and the like. Based on those principles, relaxin can be used to reduce cervical dilation in human females, preferably pregnant females, prone to premature delivery.

The state of the art allows the clinician to determine the dosage regimen of relaxin for each individual patient. As an illustrative example, the guidelines provided below for relaxin can be used as guidance to determine the dosage regimen, *i.e.,* dose schedule and dosage levels, of formulations containing pharmaceutically active relaxin administered when practicing the methods of the disclosure. As a general guideline, it is expected that the daily dose of pharmaceutically active H2 human relaxin (*e.g.,* synthetic, recombinant) is typically in an amount in a range of about 0.1 to 500 µg/kg of subject body weight per day, and more typically in a range of about 1 to 500 µg/kg of subject body weight per day. Subjects may also receive pharmaceutically active H2 human relaxin (e.g., synthetic, recombinant) in an amount in a range of about 10 to 1000 µg/kg of subject body weight per day. In one embodiment, the dosages of relaxin are 10, 30, 100 and 250 µg/kg/day. In another embodiment, these dosages result in serum concentrations of relaxin of about 3, 10, 30 and 75 ng/mL, respectively. In another embodiment, the administration of relaxin is continued as to maintain a serum concentration of relaxin of from about 0.5 to about 500ng/ml, more preferably from about 0.5 to about 300 ng/ml, and most preferably from about 3 to about 75 ng/ml. In another embodiment, relaxin is continued as to maintain a serum concentration of relaxin of from about 0.1 to about 50 ng/ml, more preferably from about 0.1 to about 30 ng/ml, and most preferably from about 1 to about 20 ng/ml, depending on the subject and condition. The relaxin administration is maintained for a specific period of time or for as long as needed to inhibit cervical dilation and preterm birth. For example, the duration of relaxin treatment can be kept at a range of about 4 hours to about 96 hours depending on the patient, and one or more optional repeat treatments as needed. Alternatively, relaxin can be administered over several weeks. For example, portable infusion pumps for subcutaneous administration of relaxin can be used to treat a female at risk for premature delivery over several weeks without requiring her to stay in the hospital.

Single or multiple administrations of relaxin formulations may be administered depending on the dosage and frequency as required and tolerated by the female who is at risk of premature delivery. The formulations should provide a sufficient quantity of relaxin to effectively reduce premature cervical dilation. A typical pharmaceutical formulation for intravenous administration of relaxin would depend on the specific therapy. For example, relaxin may be administered to a patient through monotherapy (*i.e.,* with no other concomitant medications) or in combination therapy with another medication such as progesterone, a tocolytic agent or other drug. Notably, the dosages of relaxin administered to a patient may vary depending on age, stage of pregnancy, drug tolerance, and concomitant medications and conditions.

### EXPERIMENTAL

The following specific examples are intended to illustrate the disclosure and should not be construed as limiting the scope of the claims.

### EXAMPLE 1

### Clinical Study to Assess Safety and Effect of Human Relaxin on Cervical Ripening

*Overview of the Clinical Study.* The current study was designed with two objectives. The first was to determine the safety of intravenous administration of recombinant human relaxin in women at term for 24 hours. The second objective was to determine whether a high dose of relaxin administered by this method for 24 hours could ripen the cervix or induce labor. The primary efficacy endpoint was change from baseline in Bishop score, which is a composite, well accepted measurement of cervical ripening (Bishop, 1964). The score has 5 components, all of which contribute to the cervical changes necessary for vaginal delivery of the baby: dilation, effacement, station, consistency and position of cervix. Surprisingly, relaxin did not advance cervical ripening or labor onset, but in contrast reduced cervical dilation in these subjects.

*Study Subjects.* Study subjects were healthy primiparous women at ≥ 40 weeks gestation who were admitted to the hospital for scheduled induction of labor. The inclusion and exclusion criteria for study subjects are listed in Table 1. This study was conducted in compliance with the requirements of the protocol, International Conference on Harmonization (ICH), Good Clinical Practices (GCP), FDA, local regulations and BAS Medical, Inc.'s standard operating procedures (SOP). The investigators were responsible for obtaining informed consent from each patient participating in the study. All pertinent aspects of the study were explained to the patient before he or she signed the informed consent form. Informed consent was approved by local ethics committees and was obtained from the patient before any activity or treatment was undertaken which was not part of routine care. This included, but was not limited to, the performance of diagnostic or therapeutic procedures and the administration of the first dose of the study medication.

**Table 1 Study Subjects**

| ***Key Inclusions*** |
|---|
| Healthy, primiparous women > 40 weeks gestation |
| Bishop score < 4 |
| Intact membranes, vertex presentation of fetus and less than 8 uterine contractions per hour |
| Reactive fetal nonstress test |
| Singleton pregnancy |
| 18 - 40 years old |
| Pre-pregnancy BMI < 29 and weight gain < 18 kg during pregnancy |

| ***Key Exclusions*** |
|---|
| Hemoglobin < 8.5 gm/dL |
| Current diagnosis of hypertension (sustained SBP > 140 mmHg or DBP > 90 mmHg) |
| Diagnosis of gestational toxicosis (manifested by sickness, vomiting, edema and/or proteinuria) |
| Weight < 50 kg |
| Maternal chronic renal disease |
| Maternal autoimmune or collagen vascular disease |
| Maternal pre-gestational diabetes or gestational diabetes requiring insulin therapy |
| History of endometriosis |
| Electrocardiographic evidence of any cardiac conduction abnormality |
| Severe maternal cardiac disease requiring ICU monitoring |
| Planned and/or prior caesarean delivery, or prior classical uterine incision |
| Placenta or vasa previa |
| Malpresentation of fetus, including transverse lie |
| Prolapsed umbilical cord |
| Active genital herpes infection |
| Known fetal anomaly |
| Intrauterine growth restriction (IUGR); weight below the 10th percentile for gestational age and |
| whose abdominal circumference is below the 2.5th percentile |

*Study Design:* The study was conducted in two parts:
Part A was a multicenter, randomized, double-blind, placebo-controlled, dose escalation study of 18 subjects at ≥ 40 weeks gestation and who were scheduled for induction. Doses of 7.5, 25 and 75 mcg/kg/day of relaxin were tested. Subjects were treated in cohorts of 6 and randomly assigned to receive relaxin or placebo in a 4:2 ratio, respectively. Dose escalation to the next dose level occurred once safety data was reviewed.

Eligible patients were admitted to the hospital at least 24-hours prior to scheduled induction. Either relaxin or matching placebo was administered by continuous intravenous infusion for 24 hours prior to induction. Subjects were monitored throughout the administration of study medication for blood pressure and heart rate, as well as for uterine contractions and the onset of labor. Fetal nonstress testing and heart rate monitoring were conducted for evaluation of fetal well-being. Blood samples were drawn for serum chemistry and hematology at baseline, 12 and 24 hr of dosing, and at 2 days, 1 week and 4 weeks after delivery. Samples for the evaluation of serum relaxin levels were drawn at baseline, 30 minutes, 1, 4, 6, 12, and 24 hr of dosing and a sample of cord blood was also drawn for relaxin measurement. A sample was also drawn from the study subject at 2 days post-delivery. Samples for measurement of development of anti-relaxin antibodies were drawn prior to dosing and at 1 and 4 weeks post-delivery. Neonates were evaluated for relaxin levels at 2 days and for the presence of anti-relaxin antibodies at 1 and 4 weeks.

Part B of the study was a multi-center, randomized, double-blind, placebo-controlled, parallel-group study of 50 subjects at ≥ 40 weeks gestation and who were scheduled for induction. Subjects were randomized in a 1:1 ratio to the most appropriate dose of relaxin determined from Part A or to placebo. As in Part A, eligible subjects were administered an intravenous infusion of relaxin or placebo for 24 hours prior to induction. Subjects and neonates were evaluated in a manner identical to that in Part A for all assessments.

The primary efficacy endpoint was change from baseline in Bishop score at 6, 12 and 24 hours (or end of study drug administration). The score ranges from 0 (absence of any cervical changes) to a maximum of 13, representing a cervix that is completely dilated (>5 cm), effaced (>80%), soft, at a +1 or +2 station, and anteriorly positioned (Bishop, 1964). A number of secondary efficacy endpoints were also explored: Time to complete dilation; proportion of subjects with Bishop score change > 3; proportion of subjects with Bishop score > 5; proportion of subjects with Bishop score > 8; time to active labor; time to delivery (vaginal or c-section); frequency of uterine contractions; incidence of uterine hyperstimulation requiring terbutaline treatment or discontinuation of study drug (relaxin or placebo) infusion; incidence of spontaneous labor; rates of c-section; incidence of abnormal fetal heart rate tracings; changes from baseline in systolic and diastolic blood pressure; changes from baseline in serum creatinine and predicted creatinine clearance as determined by the Cockroft-Gault formula.

*Safety. Safety* was assessed by means of reported and observed adverse events, physical examination and 12-lead ECG findings, vital sign measurements, and the results of clinical laboratory assessments. Serum clinical chemistry samples were taken at pre-dose, 12 and 24 hours following the start of dosing, 2 days, 1 week and 4 weeks postpartum. Maternal vital signs included temperature, heart rate and blood pressure. These evaluations were performed at screening, pre-dose, during dosing, 24-48 hours post-dosing and at 2 days, 1 week and 4 weeks post-partum. Measurements during the 24-48 hours post-dosing period were taken at 28, 32, 36, 40, 44 and 48 hours. Neonatal vital signs included temperature, pulse, respiration and blood pressure. All vital signs measurements were done at delivery, 2 days, 1 week and 4 weeks post-partum.

Adverse events in study subjects were recorded according to the time of onset, i.e. dosing period (0-24 hours), 24-hours post-infusion (24-48 hours) and follow-up (> 48 hours). Adverse events reported in fetuses and in neonates were also summarized by treatment group. For laboratory tests (hematology, serum chemistry, urinalysis), vital signs, physical examination and ECGs summary statistics were provided for baseline and changes from Baseline for each scheduled time by treatment group. Post-hoc repeated measure analyses of systolic and diastolic blood pressure were also performed.

One serious adverse event, cephalopelvic disproportion, was reported in a subject in the placebo group. The event occurred in the post-dosing follow-up period (> 48 hours post-dosing). No action or medication was required and the subject recovered. Serious adverse events were reported in 3 fetuses. Two events were reported in the pooled relaxin group (fetal hypoxia of moderate severity). No action or medication was required in either case and the adverse events were not related to the study drug. There was one report of acute fetal distress of moderate severity in the placebo group. Medication was administered and the fetus recovered from the event. Serious adverse events were reported in 8 neonates; one in the 7.5 mcg/kg/d dose group, 4 in the pooled relaxin and 3 in the placebo group. None were related to the study drug and all neonates recovered from the adverse event. These events included 2 reports of hemolytic disease and one report each of severe neonatal asphyxia, deficient body weight gain, macrostomia, hyperbilirubinemia, cerebral ischemia, lack of adequate spontaneous breathing, congential heart disease, meconium aspiration, and conjunctivitis.

*Statistical Analysis.* Analyses were conducted on the Intent-to-treat (ITT) population, which consisted of all randomized subjects and on the Per-protocol (PP) population, which consisted of all randomized subjects who received study medication for at least 18 hours (75% compliance) and had baseline and 24 hour (or end of treatment) assessments of the Bishop Score. For efficacy comparisons, the patients from Part A that received the active dose administered in Part B are pooled with the patients from Part B (pooled relaxin group) as are all placebo patients from parts A and B (pooled placebo group).

*Relaxin ELISA.* The measurement of serum relaxin concentrations is based on an enzyme immunoassay technique using 96-well microtiter plates coated with affinity purified goat-anti-relaxin antibodies. Pre-diluted controls and unknown samples were pipetted into the wells of the microtiter plate and incubated at 2-8°C allowing for any relaxin present to bind to the anti-relaxin antibodies. After an overnight incubation the plates were washed to remove any non-reactive serum components. An affinity purified rabbit anti-relaxin peroxidase conjugate was added and allowed to incubate for 3 hours at room temperature with shaking. The added conjugate recognizes any bound relaxin. The unbound protein and reagents were removed by another wash step and followed by the addition of a substrate, tetramethylbenzidine (TMB) solution, to the wells for color development. After a 20-minute incubation, an aliquot of 2M sulfuric acid was added to stop the color reaction and the absorbance was measured at 450 nm (reference 650 nm) using a plate spectrophotometer. The intensity of the color produced was proportional to the concentration of relaxin in the sample. The relaxin levels were quantified according to a standard curve generated by measuring purified recombinant relaxin in a 20% human serum matrix utilizing a four-parameter curve fit equation. This assay had a working sensitivity of 96 pg/ml. The controls spiked with relaxin at a low, mid, and high level were analyzed in this ELISA had an acceptable between-run precision, ranging from 7.21% to 9.16% coefficient of variation (CV). This study was conducted in compliance with Food and Drug Administration (FDA) Good Laboratory Practice (GLP) regulations 21 CFR, Part 58.

*Anti-Relaxin Antibody Test.* This assay is based on the enzyme immunoassay technique using 96-well microtiter plates coated with relaxin molecules. Pre-diluted controls and unknown samples were pipetted into the wells of the microtiter plate and incubated with shaking at room temperature allowing any anti-relaxin antibodies present to bind to the relaxin. After a three-hour incubation the plate was washed to remove any non-reactive serum components. A species-specific anti-IgG/IgM horseradish peroxidase conjugate which recognizes any IgG or IgM antibodies was bound to the relaxin solid phase. The unbound protein and reagents were removed by another wash step followed by the addition of a substrate, tetramethylbenzidine (TMB) solution. After a ten minute incubation, an aliquot of 2M sulfuric acid was added and the absorbance was measured at 450 nm (reference 630 nm) using a plate spectrophotometer. The intensity of the color produced was proportional to the concentration of anti-relaxin antibodies in the sample. Antibody-positive samples were determined by comparing the optical density with a predetermined cutoff optical density.

*Findings.* A total of 72 subjects (22 in Part A and 50 in Part B), of which 40 were treated with relaxin and 32 with placebo, were enrolled into the study. The average age of the study population was 24 years (range: 18 - 32 years). The majority of subjects were Caucasian (85%, n = 61). Subjects presented with an average pre-pregnancy weight of 60 kg (range: 45 - 80 kg) and were at 40 weeks' gestation. Demographics were similar among the subjects randomized to the 3 dose levels in Part A and to the 2 dose groups in Part B. All subjects presented with a Bishop score < 4 both at screening and at the pretreatment assessment. The average Bishop score among all subjects was 2.1 + 1.5 (mean + SD) at both screening and at the pre-dosing baseline measurement.

Part A: Seven subjects received 7.5 µg/kg/day relaxin and 3 subjects received placebo in the first cohort of Part A. The safety data from these subjects were reviewed in blinded fashion and found to be acceptable, so six subjects were randomized to Cohort 2 and enrolled to receive 25 mcg/kg/day relaxin or placebo. Once safety in these subjects was affirmed, enrollment in the third cohort occurred and four and two subjects were dosed with 75 mcg/kg/day relaxin and placebo, respectively. Based on the data from the 22 subjects in Part A, the 75 mcg/kg/day relaxin dose was selected as the dose for study in Part B.

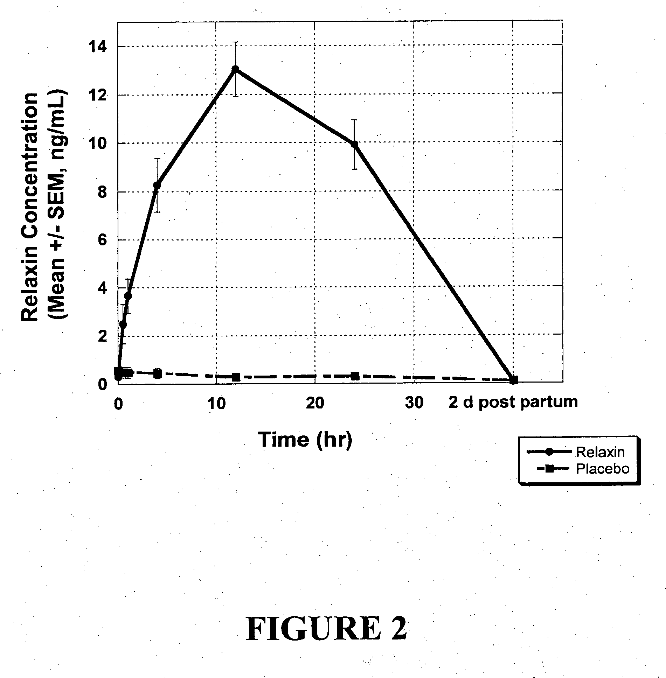

Part B: A total of 50 patients, equally distributed between the 75 mcg/kg/day and placebo groups, were enrolled into this segment of the study. A total of 9 of the 72 subjects received less than 18 hours of study drug infusion. Infusion was interrupted briefly in 4 subjects due to technical problems with the infusion pump delivery system and terminated in 16 subjects due to onset of labor (n=12) or rupture of membranes (n=4). The mean treatment exposure in the pooled relaxin group was 22.3 + 4.2 hr (n = 29) and the mean treatment exposure in the pooled placebo group was 21.4 + 6.1 hr (n = 32). Mean serum concentrations at baseline in the relaxin and placebo groups were 0.293 and 0.561 ng/mL, reflecting endogenous relaxin levels. Relaxin concentration in the relaxin group rapidly rose to a peak of 13.0 ng/mL at 12 hours of dosing, while levels in the placebo group remained constant. By 2 days post partum, concentrations in both groups dropped to levels just above levels of detection (see Figure 2).

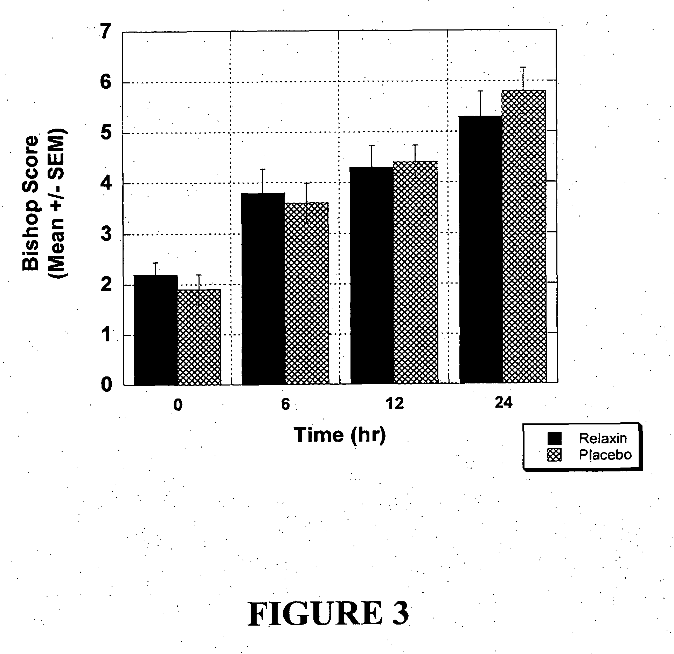

Mean Bishop scores in the pooled relaxin and placebo groups at baseline and at 6, 12, and 24 hours of study drug treatment are depicted in Figure 3. At baseline the average Bishop scores were 2.2 and 1.9 in the pooled relaxin and placebo groups, respectively. Table 2 below summarizes the Bishop scores at baseline and the changes from baseline at 6, 12 and 24 hours in the pooled placebo and pooled relaxin groups. Both treatment groups showed increases in Bishop score from baseline at 6, 12 and 24 hours of study drug infusion. The average increase ranged from 1.55 to 3.26 in the pooled relaxin group and from 1.77 to 4.19 in the placebo group.

There were no statistically significant differences between the pooled relaxin and placebo groups at any of the observation times. Individual components of the Bishop score were also similar between the pooled relaxin and pooled placebo groups except for one parameter, cervical dilation.

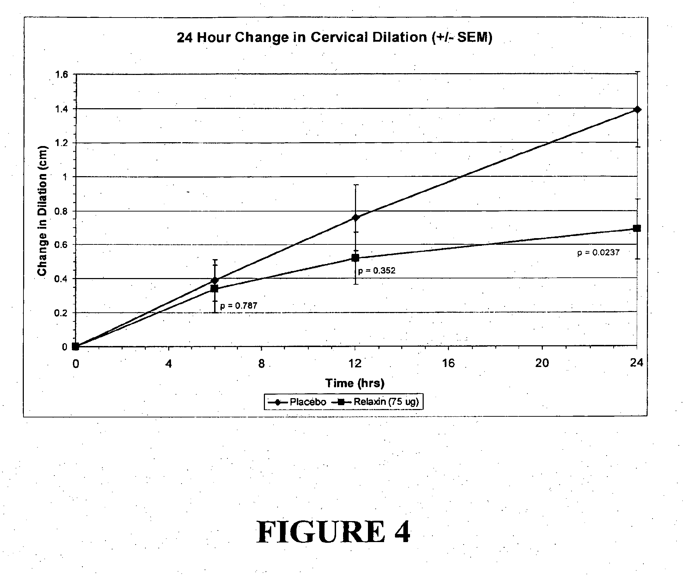

The pooled placebo and the pooled relaxin group started at the same extent of cervical dilation, and changes in dilation were measured over time (Figure 4). The placebo group showed a continuous increase in cervical dilation, changing by 1.39 cm from the initial dilation at the 24 hour time point. In contrast, the relaxin group showed only a slight increase during the first hours of relaxin administration, which leveled off over time. At the 24 hour time point cervical dilation was increased by only 0.69 cm from the initial dilation in this group. Thus, cervical dilation was significantly reduced in the relaxin group compared to the placebo group (p < 0.024 by t-test), indicating that relaxin arrests cervical dilation. Total Bishop scores in the lower relaxin dose groups (7.5 and 25µg/kg/day) tested in Part A were also no different than that seen in the pooled placebo group (data not shown). Secondary endpoints related to delivery are shown in Table 3. Relaxin infusion did not significantly affect incidence or time to vaginal delivery, incidence of spontaneous labor or time to the onset of labor. There was no effect of relaxin treatment on fetal heart rate.

**Table 2 Total Bishop Score - Change from Baseline**

| **Change from Baseline to** | **Parameter** | **75 µ/kg/d (n = 29)** | **Pooled Placebo** | **P-value *** |
|---|---|---|---|---|
| 6 hours | N | 29 | 30 | |
| | Mean | 1.55 | 1.77 | 0.2407 |
| | SD | 2.13 | 1.45 | |
| | Median | 1.00 | 2.00 | |
| | Minimum | 0 | 0 | |
| | Maximum | 9 | 4 | |
| 12 hours | N | 28 | 28 | |
| | Mean | 2.11 | 2.64 | 0.1085 |
| | SD | 2.02 | 1.64 | |
| | Median | 2.00 | 3.00 | |
| | Minimum | 0 | 0 | |
| | Maximum | 7 | 7 | |
| 24 hours | N | 23 | 26 | |
| | Mean | 3.26 | 4.19 | 0.2507 |
| | SD | 2.26 | 1.90 | |
| | Median | 4.00 | 4.00 | |
| | Minimum | 0 | 0 | |
| | Maximum | 7 | 8 | |
| Last Obs. | N | 29 | 30 | |
| | Mean | 3.59 | 4.03 | 0.4900 |
| | SD | 2.50 | 1.90 | |
| | Median | 4.00 | 4.00 | |
| | Minimum | 0 | 0 | |
| | Maximum | 9 | 8 | |

Notes on Table 2. 1) Pooled relaxin group (75 µg/kg/d) combined subjects who were taking the same active dose in Part A and B. Pooled placebo group combined subjects who were taking placebo in Part A and B. 2) Subjects who randomized in the study (ITT population) were included in this table. 3) The total Bishop Scores reported on the CRFs were used in this table. If the total Bishop score was > 13, then '13' was used. 4) Baseline value was defined as the total bishop score at pre-dose. If pre-dose value was not available, then screening value was used. 5) Last Obs. was defined as the last available value post-baseline. 6) Change was calculated as Visit minus Baseline. *P-value was obtained to assess the difference between the pooled relaxin group and pooled placebo group only.

**Table 3 Secondary Efficacy Endpoints Related to Delivery in the Pooled Relaxin and Placebo Groups**

| | **Relaxin** | **Placebo** | **p value** |
|---|---|---|---|
| Incidence of Vaginal Deliveries | 20/29 (69%) | 27/32( 84%) | 0.153 |
| Time to Vaginal Delivery (mean + SD) | 62 ± 40 hr (n = 20) | 54±29hr (n = 27) | 0.644 |
| Time to Delivery for Vaginal + C-section (mean + SD) | 69 ± 40 hr (n = 29) | 55 ± 30 hr (n = 32) | 0.251 |
| Incidence of Spontaneous Labor | 12/29 (41%) | 20/32 (63%) | 0.213 |
| Time to Onset of Active Labor | 53 hr (n = 26) | 46 hr (n = 32) | 0.412 |

*Conclusion.* The inventor's aim of the study was to determine safety of recombinant human relaxin when administered intravenously to pregnant human females and to investigate the effect of human relaxin on cervical ripening. The study reports the first use of intravenously administered human relaxin for this indication and demonstrates that over the entire dosage range, relaxin showed no relevant adverse effects. In contrast to relaxin's effect in animals, in pregnant human women at term relaxin did not result in an increase in Bishop score, the standard measurement for cervical ripening. Instead this study shows that administration of relaxin reduced the rate of cervical dilation in these women. Thus, relaxin can be used to reduce premature cervical dilation, indicating that it is highly applicable as a treatment to reduce the risk of premature delivery or miscarriage.

### EXAMPLE 2

### Clinical Study to Determine Efficacy of Relaxin in Females at Risk of Premature Delivery

*Study Overview and Design.* A randomized, double-blind, placebo controlled study is conducted to assess the efficacy of human relaxin in arresting further cervical dilation in females with preterm cervical dilation. Women with this indication are usually admitted to the hospital and are given bed rest. The study will enroll these individuals and administer relaxin by either intravenous infusion at the hospital or by using infusion pumps for subcutaneous administration, which does not require hospitalization. The primary efficacy endpoint is the extension of time from the onset of premature cervical dilation and relaxin or placebo administration to delivery. This time period will be compared between relaxin treated and control groups and statistical analysis performed.

## Claims

1. A pharmaceutically active H2 relaxin for use in a method of treating premature cervical dilation, comprising administering the relaxin to a pregnant human female.

2. Use of a pharmaceutically active H2 relaxin for the manufacture of a medicament for treatment of premature cervical dilation, comprising administering the relaxin to a pregnant human female.

3. The pharmaceutically active H2 relaxin for use according to any of claims 1-2, wherein the pregnant human female experiences symptoms selected from the group consisting of early contractions, cramps in her lower abdomen, low dull pain in her back, pressure in her pelvic area, stomach cramps, vaginal discharge, vaginal bleeding and vaginal watery fluid leakage.

4. The pharmaceutically active H2 relaxin for use according to any of claims 1-2, administered in an amount within a range of about 0.1 to 500 µg/kg of subject body weight.

5. The pharmaceutically active H2 relaxin for use according to any of claims 1-2, administered in an amount so as to maintain a serum concentration of relaxin of from about 0.5 to 50 ng/ml.

6. The pharmaceutically active H2 relaxin for use according to any of claims 1-2, administered at the beginning of the first, the beginning of the second or the beginning of the third trimester of pregnancy.

7. The pharmaceutically active H2 relaxin for use according to any of claims 1-2, administered until the onset of labor.

## Patentansprüche

1. Pharmazeutisch aktives H2-Relaxin zur Verwendung in einem Verfahren zur Behandlung von vorzeitiger Zervixdilatation, das die Verabreichung des Relaxins an einen schwangeren weiblichen Menschen umfasst.

2. Verwendung eines pharmazeutisch aktiven H2-Relaxins für die Herstellung eines Medikaments zur Behandlung von vorzeitiger Zervixdilatation, das Verabreichen des Relaxins an einen schwangeren weiblichen Menschen umfasst.

3. Pharmazeutisch aktives H2-Relaxin zur Verwendung nach einem der Ansprüche 1-2, wobei der schwangere weibliche Mensch Symptome empfindet, die aus der Gruppe bestehend aus frühen Wehen, Krämpfen in ihrem Unterleib, leichte dumpfe Schmerzen in ihrem Rücken, Druck in ihrem Beckenbereich, Magenkrämpfe, vaginalem Ausfluss, vaginalen Blutungen und vaginalem Austritt einer wässrigen Flüssigkeit ausgewählt sind.

4. Pharmazeutisch aktives H2-Relaxin zur Verwendung nach einem der Ansprüche 1-2, das in einer Menge in einem Bereich von etwa 0,1 bis 500 µg/kg Körpergewicht des Subjekts verabreicht wird.

5. Pharmazeutisch aktives H2-Relaxin zur Verwendung nach einem der Ansprüche 1-2, das in einer Menge verabreicht wird, um eine Serumkonzentration von Relaxin von etwa 0,5 bis 50 ng/ml beizubehalten.

6. Pharmazeutisch aktives H2-Relaxin zur Verwendung nach einem der Ansprüche 1-2, das zu Beginn des ersten, zu Beginn des zweiten oder zu Beginn des dritten Trimesters der Schwangerschaft verabreicht wird.

7. Pharmazeutisch aktives H2-Relaxin zur Verwendung nach einem der Ansprüche 1-2, das bis zum Einsetzen der Geburtswehen verabreicht wird.

## Revendications

1. Relaxine H2 pharmaceutiquement active destinée à être utilisée dans un procédé de traitement de la dilatation prématurée du col, comprenant l'administration de la relaxine à une femme enceinte.

2. Utilisation d'un relaxine H2 pharmaceutiquement active pour la fabrication d'un médicament pour le traitement de la dilatation prématurée du col, comprenant l'administration de la relaxine à une femme enceinte.

3. Relaxine H2 pharmaceutiquement active destinée à être utilisée selon l'une quelconque des revendications 1 et 2, la femme enceinte présentant des symptômes choisis dans le groupe consistant en contractions précoces, crampes dans le bas de l'abdomen, faible douleur diffuse dans le dos, pression dans la région pelvienne, crampes de l'estomac, perte vaginale, saignement vaginal et écoulement vaginal aqueux.

4. Relaxine H2 pharmaceutiquement active destinée à être utilisée selon l'une quelconque des revendications 1 et 2, administrée en une quantité comprise dans la plage d'environ 0,1 à 500 µg/kg de poids corporel du sujet.

5. Relaxine H2 pharmaceutiquement active destinée à être utilisée selon l'une quelconque des revendications 1 et 2, administrée en une quantité permettant de maintenir une concentration sérique de relaxine comprise entre environ 0,5 et 50 ng/ml.

6. Relaxine H2 pharmaceutiquement active destinée à être utilisée selon l'une quelconque des revendications 1 et 2, administrée au début du premier trimestre, au début du deuxième trimestre ou au début du troisième trimestre de la grossesse.

7. Relaxine H2 pharmaceutiquement active destinée à être utilisée selon l'une quelconque des revendications 1 et 2, administrée jusqu'au déclenchement du travail.
